# EUROPEAN PATENT APPLICATION

(11) **EP 4 537 766 A1**
(43) Date of publication of application: **16.04.2025**
(21) Application number: 22945946.6
(22) Date of filing: 11.08.2022
(51) Int. Cl.: A61B 8/08, A61B 8/00, G06T 15/08, G06T 7/11, G06F 3/048

(54) **ULTRASOUND DIAGNOSTIC APPARATUS AND METHOD FOR OPERATING SAME**

(30) Priority: 07.06.2022 KR 20220068844
(71) Applicant: Samsung Medison Co., Ltd., Hongcheon-gun, Gangwon-do 25108 (KR)
(72) Inventor: KIM, Han Jun, Hongcheon-gun, Gangwon-do 25108 (KR); KIM, Han Eol, Hongcheon-gun, Gangwon-do 25108 (KR); LEE, Jin Yong, Hongcheon-gun, Gangwon-do 25108 (KR); CHOI, Sung Jin, Hongcheon-gun, Gangwon-do 25108 (KR)
(74) Representative: Lorenz & Kollegen
(86) International application number: PCT/KR2022/012042
(87) International publication number: WO 2023/238987

(57) **Abstract**

An ultrasound diagnostic apparatus may include an ultrasound signal transceiving module configured to transmit an ultrasound signal to an object and receive an ultrasound echo signal reflected from the object, a controller configured to extract area information that is information about an area in the object occupied by each of the plurality of object bodies included in the object, based on the echo signal received from the ultrasound signal transceiving module, and generate a plurality of pieces of preset data corresponding to each of the plurality of object bodies by changing visibility-related parameters corresponding to an area occupied by each of the plurality of object bodies according to a predetermined value, based on the area information, a parameter information storage portion configured to store information about the predetermined value and provide the information about the predetermined value to the controller in response to a request by the controller, and a display configured to receive the plurality of pieces of preset data from the controller and render and output preset three-dimensional volume data of the object based on the plurality of pieces of preset data.

## Description

### Technical Field

The present disclosure relates to an ultrasound diagnostic apparatus and an operation method thereof, and particularly, to an ultrasound diagnostic apparatus which may facilitate observation of an object body that a user desires among a plurality of object bodies, by dividing an object into a plurality of object bodies and generating three-dimensional volume data for each of the object bodies, and an operation method thereof.

### Background Art

Recently, in the medical field, various medical imaging devices are widely used to image and obtain information about the biological tissues of the human body for the purpose of early diagnosis or operation of various diseases. Representative examples of these medical imaging devices may include ultrasound diagnostic apparatuses, CT apparatuses, and MRI apparatuses.

An ultrasound diagnostic apparatus emits to an object an ultrasound signal generated from a transducer of a probe and receives information of an echo signal reflected from the object, thereby obtaining an image of an internal portion of the object. In particular, the ultrasound diagnostic apparatus is used for medical purposes, such as observation of the inside of an object, detection of foreign materials, and injury measurement, and the like. The ultrasound diagnostic apparatus has the advantages of having higher stability than diagnostic apparatuses that use X-rays, being capable of displaying images in real time, and being safe due to no radiation exposure, which leads to a wide use of the ultrasound diagnostic apparatus with other burn diagnostic apparatuses.

### Disclosure

### Technical Problem

The technical objective to solve is to provide an ultrasound diagnostic apparatus which may facilitate observation of an object body desired by a user among a plurality of object bodies included in an object, and an operation method thereof.

### Technical Solution

An ultrasound diagnostic apparatus according to an embodiment of the present disclosure may segment a plurality of object bodies in an ultrasound image of an object and change visibility-related parameters of each of a plurality of segmented object bodies, thereby facilitating observation of an object body desired by a user among the plurality of object bodies.

### Advantageous Effects

An ultrasound diagnostic apparatus according to an embodiment of the present disclosure may adjust visibility parameters of each of a plurality of object bodies included in an object, thereby facilitating observation of an object body desired by a user among the plurality of object bodies.

### Description of Drawings

FIG. 1 is a block diagram of a configuration of an ultrasound diagnostic apparatus according to an embodiment.
(a) to (c) of FIG. 2 are views showing an ultrasound diagnostic apparatus according to an embodiment.
FIG. 3 is a view showing an example of an ultrasound diagnostic apparatus displaying an ultrasound image of an object including a plurality of object bodies.
FIG. 4 is a view for explaining content in which an ultrasound diagnostic apparatus classifies a plurality of object bodies included in an object in units of classes, according to an embodiment.
FIG. 5 is a flowchart of a method in which an ultrasound diagnostic apparatus according to an embodiment outputs a three-dimensional volume image of an object including a plurality of object bodies.
FIG. 6 is a block diagram for explaining a configuration and operation of an ultrasound diagnostic apparatus according to an embodiment.
FIG. 7 is a block diagram for explaining a configuration and operation of an ultrasound diagnostic apparatus according to another embodiment.
FIGS. 8A and 8B are tables for explaining a class information storage portion according to an embodiment.
FIG. 9 is a flowchart for explaining a parameter information storage portion according to an embodiment.
FIG. 10 is a block diagram for explaining a configuration and operation of an ultrasound diagnostic apparatus according to another embodiment.
FIG. 11 is a block diagram for explaining a user interface according to an embodiment. FIGS. 12A, 12B, and 12C are views for explaining content displayed on a display of an ultrasound diagnostic apparatus according to an embodiment.
FIG. 13 is a flowchart for explaining content in which an ultrasound diagnostic apparatus according to an embodiment outputs an ultrasound image that reflects changes.

### Best Mode

According to an embodiment of the present disclosure, an ultrasound diagnostic apparatus may include an ultrasound signal transceiving module configured to transmit an ultrasound signal to an object and receive an ultrasound echo signal reflected from the object, a controller configured to extract area information that is information about an area in the object occupied by each of the plurality of object bodies included in the object, based on the echo signal received from the ultrasound signal transceiving module, and generate a plurality of pieces of preset data corresponding to each of the plurality of object bodies by changing visibility-related parameters corresponding to an area occupied by each of the plurality of object bodies according to a predetermined value, based on the area information, a parameter information storage portion configured to store information about the predetermined value and provide the information about the predetermined value to the controller in response to a request by the controller, and a display configured to receive the plurality of pieces of preset data from the controller and render and output preset three-dimensional volume data of the object based on the plurality of pieces of preset data.

According to an embodiment, the controller may be further configured to generate the three-dimensional volume data of the object when the echo signal is received, extract area information corresponding to each of the plurality of object bodies from the three-dimensional volume data of the object, by using a semantic segmentation method, perform a classification operation to match each of the plurality of object bodies to any one of a plurality of predetermined classes, and generate a plurality of pieces of preset data corresponding to each of the plurality of object bodies, based on the extracted area information corresponding to each of the plurality of object bodies.

According to an embodiment, the plurality of predetermined classes may correspond to any one of a plurality of organs included in a human body or a fetus, and the controller may include a class information storage portion including information about the plurality of predetermined classes and information about a plurality of organs and a fetus corresponding to each of the plurality of predetermined classes.

According to an embodiment, the parameter information storage portion may include information about the plurality of classes and information about the predetermined value corresponding to each of the plurality of classes.

According to an embodiment, the visibility-related parameters may include at least any one of color, opacity, and texture, and the parameter information storage portion may be further configured to store information about a predetermined value corresponding to each of the visibility-related parameters for each class.

According to an embodiment, the ultrasound diagnostic apparatus may further include a user interface configured to change the visibility-related parameters, the user interface may include at least any one of a color adjustment portion, an opacity adjustment portion, and a texture adjustment portion, and when changes to the visibility-related parameters are input through the user interface, the controller may be further configured to change visibility-related parameters for pieces of preset data corresponding to each of the plurality of object bodies according to the changes and provided pieces of changed data to the display.

According to an embodiment, the user interface may further include a class selection portion configured to select any one of the plurality of classes, wherein, when changes to the visibility-related parameters are input through the user interface, the controller may be further configured to change visibility-related parameters included in pieces of preset data of the plurality of object bodies corresponding to a class selected by the class selection portion among the plurality of object bodies according to a user's input.

According to an embodiment, the display may be further configured to, when receiving the pieces of changed data from the controller, render and output the three-dimensional volume data of the object changed based on the changed data.

According to an embodiment, the controller may further include a data storage portion configured to store a plurality of pieces of preset data corresponding to each of the plurality of object bodies, and when receiving the changes from the user interface, may be further configured to receive the plurality of pieces of preset data from the data storage portion and change visibility-related parameters included in the plurality of pieces of preset data received from the data storage portion, according to the changes.

According to an embodiment, the controller may be further configured to match each of the plurality of object bodies to any one of the plurality of predetermined classes, based on the information about the plurality of predetermined classes and the information about a plurality of organs and a fetus corresponding to each of the plurality of predetermined classes included in the class information storage portion.

An operation method of an ultrasound diagnostic apparatus, according to the present disclosure, may include transmitting an ultrasound signal to an object and receiving an ultrasound echo signal reflected from the object, extracting area information that is information about an area in the object occupied by each of the plurality of object bodies included in the object, based on the echo signal, changing visibility-related parameters corresponding to an area occupied by each of the plurality of object bodies according to a predetermined value based on the area information and generating a plurality of pieces of preset data corresponding to each of the plurality of object bodies, and rendering and outputting preset three-dimensional volume data of the object based on the plurality of pieces of preset data.

According to an embodiment, the operation method may further include storing information about the predetermined value in a parameter information storage portion included in the ultrasound diagnostic apparatus.

According to an embodiment, the extracting of the area information may include generating the three-dimensional volume data of the object based on the echo signal, extracting area information corresponding to each of the plurality of object bodies by using a semantic segmentation method, matching each of the plurality of object bodies to any one of a plurality of predetermined classes, based on the extracted area information corresponding to each of the plurality of object bodies, and generating a plurality of pieces of preset data corresponding to each of the plurality of object bodies.

According to an embodiment, the plurality of predetermined classes may correspond to any one of a plurality of organs included in a human body or a fetus, the operation method may further include storing information about the plurality of classes and information about a plurality of organs and a fetus corresponding to each of the plurality of predetermined classes in a class information storage portion in the ultrasound diagnostic apparatus.

According to an embodiment, the storing of the information about the predetermined value in the parameter information storage portion may include storing information about the predetermined value corresponding to each of the plurality of classes.

According to an embodiment, the visibility-related parameters may include at least any one of color, opacity, and texture, and the storing of the information about the predetermined value in the parameter information storage portion included in the ultrasound diagnostic apparatus may include string information about a predetermined value corresponding to each of the visibility-related parameters for each class.

According to an embodiment, the operation method may further include, when changes to the visibility-related parameters are input from outside, changing visibility-related parameters with respect to pieces of preset data corresponding to each of the plurality of object bodies, according to the changes, and rendering and outputting three-dimensional volume data of the object changed based on the changed data.

According to an embodiment, the changing of the visibility-related parameters may further include selecting any one of the plurality of classes according to a user's input, and changing visibility-related parameters included in pieces of preset data of the plurality of object bodies corresponding to a class selected from among the plurality of object bodies.

According to an embodiment, the operation method may further include storing a plurality of pieces of preset data corresponding to each of the plurality of object bodies in a data storage portion included in the ultrasound diagnostic apparatus.

According to an embodiment, the matching of the each of the plurality of detected object bodies to any one of a plurality of predetermined classes may include matching each of the plurality of object bodies to any one of the plurality of predetermined classes, based on the information about the plurality of predetermined classes and the information about a plurality of organs and a fetus corresponding to each of the plurality of predetermined classes stored in the class information storage portion.

The effects of the present disclosure are not limited to the above-described effects, and other various effects that are not described in the disclosure may be clearly understood from the following descriptions and accompanying drawings by one skilled in the art to which the present disclosure belongs.

### Mode for Invention

The present specification describes the principle of the disclosure and discloses embodiments to clarify the scope of rights of the disclosure and enable one skilled in the art to which the disclosure pertains to work the disclosure. The disclosed embodiments may be implemented in various forms.

In the disclosure, when a constituent element "connects" or is "connected" to another constituent element, the constituent element contacts or is connected to the other constituent element not only directly, but also indirectly, and the indirect connection includes a connection through a wireless communication network.

Furthermore, the terms used in the disclosure are merely used to describe embodiments, and are not intended to limit the disclosure. As used herein, the singular forms "a," "an," and "the" are intended to include the plural forms as well, unless the context clearly indicates otherwise. In the specification, it is to be understood that the terms such as "including," "having," and "comprising" are intended to indicate the existence of the features, numbers, steps, actions, components, parts, or combinations thereof disclosed in the disclosure, and are not intended to preclude the possibility that one or more other features, numbers, steps, actions, components, parts, or combinations thereof may exist or may be added.

Furthermore, in the specification, while such terms including an ordinal number, such as "first," "second," etc., may be used to describe various components, such components must not be limited to the above terms. The above terms are used only to distinguish one element from another. For example, without departing from the right scope of the disclosure, a first constituent element may be referred to as a second constituent element, and vice versa.

Furthermore, the terms such as "... portion," "... unit," "... block," "... member," "... module," etc. stated in the disclosure may signify a unit to process at least one function or operation. For example, the terms may mean at least one process performed by at least one piece of hardware such as a field-programmable gate array (FPGA) or an application-specific integrated circuit (ASIC), at least one piece of software stored in memory, or a processor.

A sign attached to each operation is used to identify each operation. These signs do not indicate an order of respective operations, and the respective operations may be implemented differently from the specified order unless a specific order is clearly stated in the context.

Furthermore, in the specification, an image may include a medical image obtained by a medical imaging apparatus, such as a magnetic resonance imaging (MRI) apparatus, a computed tomography (CT) apparatus, an ultrasound imaging apparatus, and an X-ray imaging apparatus.

Furthermore, in the specification, an "object," which is a target of imaging, may include a human, an animal, or a part thereof. For example, the object may include a part (an organ, etc.) of a human body or a phantom, and the like.

In the specification, an "ultrasound image" means an image of an object processed based on ultrasound signals transmitted to an object and reflected from the object.

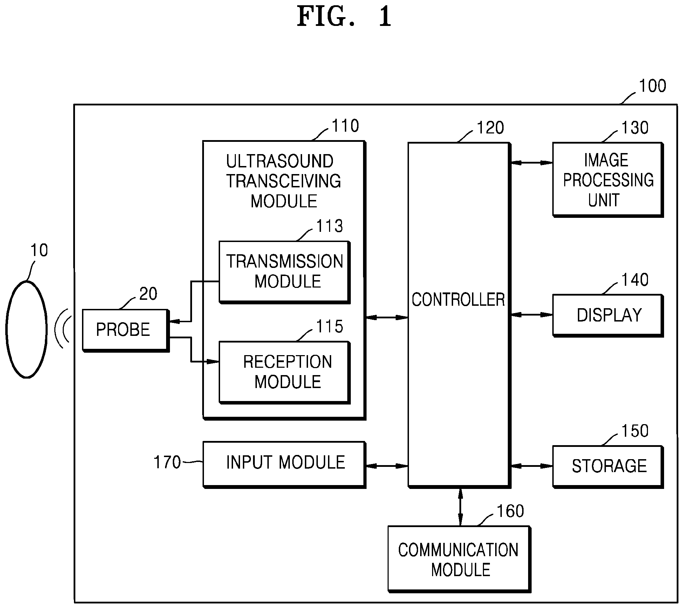

Hereinafter, an ultrasound probe and a manufacturing method thereof according to an embodiment are described in detail with reference to FIGS. 1 to 10.

FIG. 1 is a block diagram of a configuration of an ultrasound diagnostic apparatus according to an embodiment.

An ultrasound diagnostic apparatus 100 according to an embodiment may include a probe 20, an ultrasound transceiving module 110, a controller 120, an image processing unit 130, a display 140, a storage 150, a communication module 160, and an input module 170.

The ultrasound diagnostic apparatus 100 may be implemented by not only a cart type, but also a portable type. Examples of a portable type ultrasound diagnostic apparatus may include a smartphone, a laptop computer, a PDA, a tablet PC, or the like, which includes a probe and an application, but the present disclosure is not limited thereto.

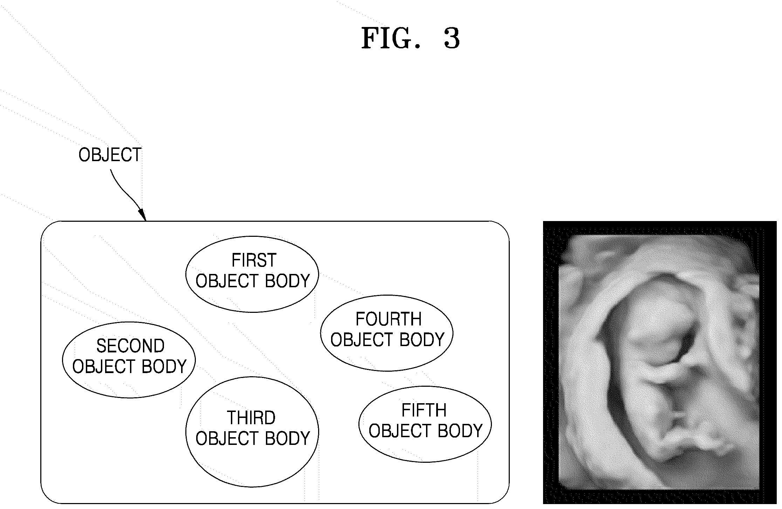

The probe 20 may include a plurality of transducers (see '1134' of FIG. 3). The plurality of transducers 1134 may transmit ultrasound signals to an object 10 according to a transmitting signal applied from a transmission portion 113. The plurality of transducers 1134 may receive the ultrasound signals reflected from the object 10 to form receiving signals. Furthermore, the probe 20 may be implemented integrally with the ultrasound diagnostic apparatus 100 or implemented as a separation type connected to the ultrasound diagnostic apparatus 100 in a wired/wireless manner. Furthermore, the ultrasound diagnostic apparatus 100 may include one or a plurality of probes 20 depending on the implementation type. According to an embodiment, the probe 20 may be referred to as an ultrasound probe 20.

The controller 120 may control the transmission portion 113 to form transmitting signals to be applied to each of the plurality of transducers 1134, considering the locations and focus points of the plurality of transducers 1134 included in the probe 20.

The controller 120 may control the transmission portion 113 to generate ultrasound data, by analog-to-digital converting the receiving signals received from the probe 20 and summing the digitally converted receiving signals, considering the locations and focus points of the plurality of transducers 1134.

The image processing unit 130 generates an ultrasound image using the ultrasound data generated by ultrasound the receiving portion 115.

The display 140 may display the generated ultrasound image and various pieces of information processed in the ultrasound diagnostic apparatus 100. The ultrasound diagnostic apparatus 100 may include one or a plurality of the displays 140 depending on implementation types. Furthermore, the display 140 may be coupled to a touch panel to be implemented as a touch screen.

The controller 120 may control the overall operation of the ultrasound diagnostic apparatus 100 and the signal flow between the internal components of the ultrasound diagnostic apparatus 100. The controller 120 may include a memory for storing a program or data to perform functions of the ultrasound diagnostic apparatus 100, and a processor for processing the program or data. Furthermore, the controller 120 may control the operation of the ultrasound diagnostic apparatus 100 by receiving a control signal from the input module 170 or an external device.

The ultrasound diagnostic apparatus 100 may include the communication module 160 and may be connected to an external device (e.g., a server, a medical device, a portable device (a smartphone, a tablet PC, a wearable device, etc.)) through the communication module 160.

The communication module 160 may include one or more components which enable communication with the external device, for example, at least one of a short-range communication module, a wired communication module, and a wireless communication module.

The communication module 160 may receive the control signal and data from the external device and transmit the received control signal to the controller 120 to allow the controller 120 to control the ultrasound diagnostic apparatus 100 according to the received control signal.

Alternatively, as the controller 120 transmits the control signal to the external device through the communication module 160, the external device may be controlled according to the control signal of the controller 120.

For example, the external device may process data of the external device according to the control signal of the controller 120 through the communication module.

A program (artificial intelligence, etc.) for controlling the ultrasound diagnostic apparatus 100 may be installed on the external device, and the program may include instructions to perform part or the whole of the operation of the controller 120.

The program may be previously installed on the external device, and may be installed as a user of the external device downloads the program from a server that provides an application. The server that provides an application may include a recording medium on which a corresponding program is stored.

Furthermore, in a system consisting of a server and a client device, the program may include a storage medium of the server or a storage medium of the client device. Alternatively, when a third device (a smartphone, a tablet PC, a wearable device, etc.) communicatively connected to the server or the client device exists, a program product may include a storage medium of the third device. Alternatively, the program may be transmitted from the server to the client device or the third device, or may include a S/W program transmitted from the third device to the client device.

In this case, a method according to the disclosed embodiments may be performed as one of the server, the client device, and the third device performs the program. Alternatively, a method according to the disclosed embodiments may be executed in a distribution method as two or more of the server, the client device, and the third device execute the program.

For example, as the server (e.g., a cloud server or an artificial intelligence server, etc.) executes the program stored in the server, a client device that is communicatively connected to the server may be controlled to perform a method according to the disclosed embodiments.

The storage 150 may store various pieces of data or a program for controlling the ultrasound diagnostic apparatus 100, ultrasound data that is input/output, the obtained ultrasound image, and the like.

The input module 170 may receive a user's input to control the ultrasound diagnostic apparatus 100. For example, the user's input may include an input to manipulate a button, a key pad, a mouse, a trackball, a jog switch, a knob, and the like, an input to touch a touch pad or a touch screen, a voice input, a motion input, a biological information input (e.g., iris recognition, fingerprint recognition, etc.), and the like, but the present disclosure is not limited thereto.

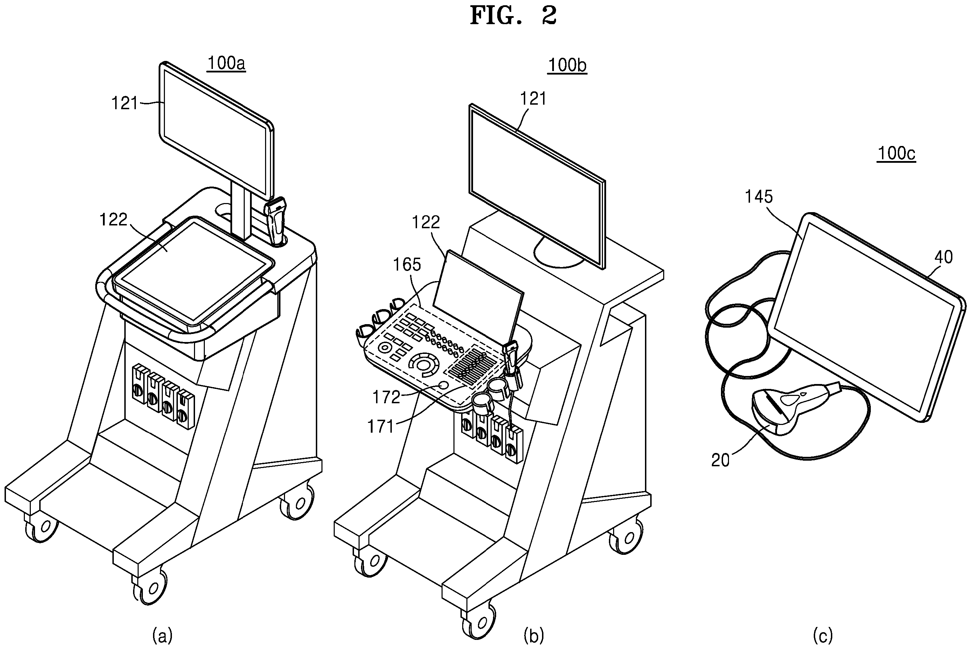

An example of the ultrasound diagnostic apparatus 100 according to an embodiment is described below with reference to (a) to (c) of FIG. 2.

(a) to (c) of FIG. 2 are views showing an ultrasound diagnostic apparatus according to an embodiment.

Referring to (a) and (b) of FIG. 2, ultrasound diagnostic apparatuses 100a and 100b may include a main display 121 and a sub-display 122. One of the main display 121 and the sub-display 122 may be implemented by a touch screen. The main display 121 and the sub-display 122 may display the ultrasound image or various pieces of information processed in the ultrasound diagnostic apparatuses 100a and 100b. Furthermore, the main display 121 and the sub-display 122 may be implemented by touch screens, may provide GUIs, and may receive from user data to control the ultrasound diagnostic apparatuses 100a and 100b. For example, the main display 121 may display an ultrasound image, and the sub-display 122 may display a control panel to control display of an ultrasound image in a GUI form. The sub-display 122 may receive an input of data to control display of an image through the control panel displayed in a GUI form. The ultrasound diagnostic apparatuses 100a and 100b may control display of the ultrasound image displayed on the main display 121 by using the received control data.

Referring to (b) of FIG. 2, the ultrasound diagnostic apparatus 100b may further include a control panel 165 in addition to the main display 121 and the sub-display 122. The control panel 165 may include a button, a trackball, a jog switch, a knop, and the like, and may receive data from a user data to control the ultrasound diagnostic apparatus 100b. For example, the control panel 165 may include a Time Gain Compensation (TGC) button 171, a Freeze button 172, and the like. The TGC button 171 is a button to set a TGC value for each depth of an ultrasound image. Furthermore, when detecting an input of the Freeze button 172 during scanning an ultrasound image, the ultrasound diagnostic apparatus 100b may maintain a state of displaying a frame image at a corresponding time point.

A button, a trackball, a jog switch, a knop, and the like included in the control panel 165 may be provided, as a GUI, to the main display 121 or the sub-display 122.

Referring to (c) of FIG. 2, an ultrasound diagnostic apparatus 100c may be implemented as a portable type. Examples of the ultrasound diagnostic apparatus 100c of a portable type may include a smartphone, a laptop computer, a PDA, a tablet PC, or the like, which includes a probe and an application, but the present disclosure is not limited thereto.

The ultrasound diagnostic apparatus 100c includes the probe 20 and a main body 40, and the probe 20 may be connected to one side of the main body 40 in a wired or wireless manner. The main body 40 may include a touch screen 145. The touch screen 145 may display an ultrasound image, various pieces of information processed by the ultrasound diagnostic apparatus 100c, a GUI, and the like.

The disclosed embodiments may be implemented in the form of a recording medium storing instructions executable by a computer. The instructions may be stored in the form of program code, and when executed by a processor, may generate a program module and perform an operation of the disclosed embodiments. The recording medium may be implemented by a computer-readable recording medium.

The computer-readable recording medium includes all type of recording media storing instructions to be deciphered by a computer. For example, the computer-readable recording medium may include read only memory (ROM), random access memory (RAM), magnetic tape, a magnetic disk, a flash memory, an optical data storage device, and the like.

FIG. 3 is a view showing an example of an ultrasound diagnostic apparatus displaying an ultrasound image of an object including a plurality of object bodies.

The ultrasound diagnostic apparatus may transmit an ultrasound signal to an object and receive an echo signal that is reflected, and generate data to output an ultrasound image of the object based on the received echo signal. The ultrasound diagnostic apparatus may output the ultrasound image of the object based on the generated data. The output image may be a two-dimensional or three-dimensional image.

The left picture of FIG. 3 is a schematic representation of an ultrasound image of an object output through the display of the ultrasound diagnostic apparatus, and the right picture of FIG. 3 is an example of an actual image displayed on the display of the ultrasound diagnostic apparatus. Referring to the left picture of FIG. 3, the object may include a plurality of object bodies (a first object body to a fifth object body) inside the object or on a surface thereof. In this case, the ultrasound images respectively corresponding to the plurality of object bodies included in the object may be displayed on one screen with the same color, texture, and opacity. For example, referring to the right picture of FIG. 3, the ultrasound diagnostic apparatus may output a three-dimensional volume image of the uterus inside the human body. In this case, a plurality of object bodies, for example, a fetus, amniotic fluid, a placenta, an umbilical cord, and the like, included in the uterus may be displayed together through an ultrasound image. In this state, ultrasound images corresponding to a uterus, and a fetus, amniotic fluid, a placenta, an umbilical cord, and the like included in the uterus may be rendered with the same color, texture, and opacity to be output on one screen. Accordingly, it may not be easy to distinguish and observe each object body.

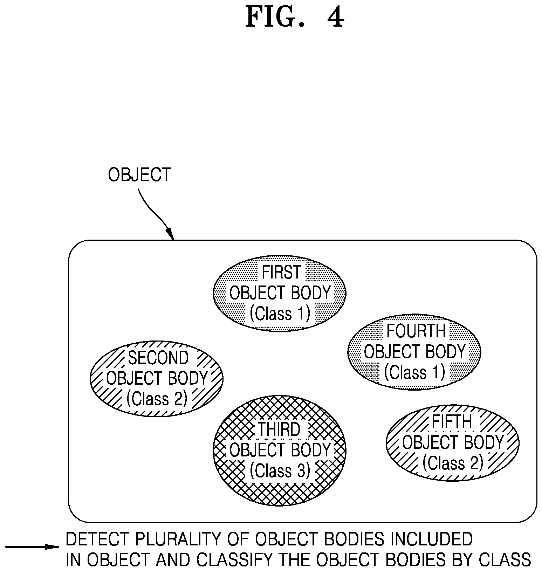

FIG. 4 is a view for explaining content in which an ultrasound diagnostic apparatus classifies a plurality of object bodies included in an object in units of classes, according to an embodiment.

The ultrasound diagnostic apparatus according to an embodiment may detect a plurality of object bodies included in the object by segmenting the same. In an embodiment, the ultrasound diagnostic apparatus may extract area information that is information about an area in the object occupied by each of the plurality of object bodies included in the object, based on an echo signal received from an ultrasound transceiving module. Each of the plurality of object bodies may be classified according to preset classes based on the extracted area information. In an embodiment, the classes may correspond to a specific part of the body, an organ, tissue, a fetus, or like classified according to clinical significance. For example, referring to FIG. 4, the object may include a plurality of object bodies (a first object body to a fifth object body), and the plurality of object bodies (the first object body to the fifth object body) may be classified into preset classes (Class1 to Class 3) according to clinical significance.

In detail, for example, in FIG. 4, the object may be 'the body of a fetus.' The body of a fetus may include a plurality of object bodies corresponding to individual body parts of the fetus. For example, the body of a fetus may include the head of a fetus, the right hand of a fetus, the left hand of a fetus, the right foot of a fetus, the left foot of fetus, and the like. In FIG. 4, the 'first object body' and the 'fourth object body' may be 'the right hand of a fetus' and 'the left hand of a fetus,' respectively. In FIG. 4, the 'second object body' may be 'the head of a fetus'. In FIG. 4, the 'second object body' and the 'fifth object body' may be 'the right foot of a fetus' and 'the left foot of fetus,' respectively. The ultrasound diagnostic apparatus according to an embodiment may set 'the hand of a fetus' to Class 1, 'the foot of a fetus' to Class 2, and 'the head of a fetus' to Class 3, and store information about each class in a separate storage portion. The ultrasound diagnostic apparatus according to an embodiment may classify the plurality of object bodies (the first object body to the fifth object body) included in the object (fetus) by corresponding classes, based on the information about each class stored in the separate storage portion.

The operation of segmenting and detecting a plurality of object bodies included in the object and then classifying the plurality of object bodies by class may be performed according to various well-known methods. In an embodiment, the operation of segmenting and detecting a plurality of object bodies from an object and the operation of classifying the plurality of object bodies by class may be performed by machine learning. Any one model of a plurality of well-known machine learning models may be used for the machine learning operation. In detail, neural network-based Convolutional Neural Networks (CNNs), Recurrent Neural Networks (RNNs), LSTM, Encoder-Decoder, Auto-Encoder, Generative Adversarial Networks (GANs) models may be used therefor. In detail, the operations of detecting a plurality of object bodies and classifying the plurality of object bodies by class may be performed by a 'semantic segmentation' method. The 'semantic segmentation' may refer to a method of segmenting an object body having a specific meaning in an object. The ultrasound diagnostic apparatus according to an embodiment may learn, based on a predetermined algorithm, to which class among a plurality of classes a specific part of the body, an organ, tissue, a fetus, or the like corresponds. The ultrasound diagnostic apparatus may segment and detect each of the plurality of object bodies from three-dimensional volume data of the object, based on a learning result. The ultrasound diagnostic apparatus may classify each of the detected plurality of object bodies by predetermined class, based on the learning result and class information stored in the separate storage portion.

The object bodies corresponding to the respective classes may be variously set according to the intension or purpose of a user using the ultrasound diagnostic apparatus. For example, in an embodiment, 'the hand of a fetus' including the left hand of a fetus and the right hand of a fetus is set to Class 1, whereas in another embodiment, 'the right hand of a fetus' and 'the left hand of a fetus' may each be set as a separate Class.

In another embodiment, the operation of segmenting a plurality of object bodies in the object by class may be manually performed by a user. For example, a user may classify and select a plurality of object bodies in the object in units of object bodies having a specific meaning, based on the image information displayed on a GUI.

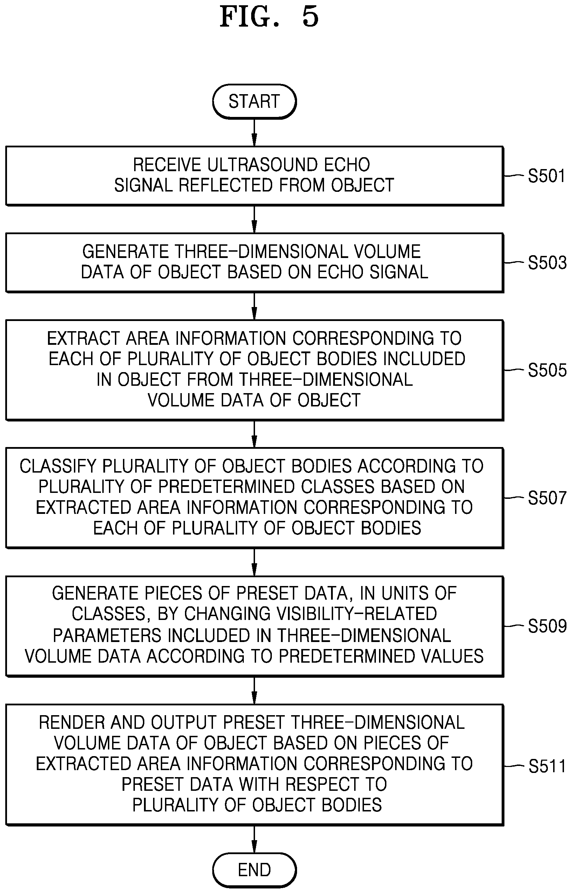

FIG. 5 is a flowchart of a method in which an ultrasound diagnostic apparatus according to an embodiment outputs a three-dimensional volume image of an object including a plurality of object bodies. In operation S501, the ultrasound diagnostic apparatus may transmit an ultrasound signal to an object and then receive a reflected echo signal. The object may include a plurality of object bodies therein.

In operation S503, the ultrasound diagnostic apparatus may generate three-dimensional volume data of the object based on the received echo signal.

In operation S505, the ultrasound diagnostic apparatus may extract area information corresponding to each of the plurality of object bodies included in the object, from the three-dimensional volume data of the object.

In operation S507, the ultrasound diagnostic apparatus may classify a plurality of object bodies according to a plurality of predetermined classes, based on the extracted area information corresponding to each of the plurality of object bodies.

In operation S509, the ultrasound diagnostic apparatus may generate pieces of preset data corresponding to each of the plurality of object bodies, in units of classes, by changing visibility-related parameters included in the three-dimensional volume data. The visibility-related parameters may refer to variables set to allow an ultrasound image output on the display of the ultrasound diagnostic apparatus to be well recognized by a user. For example, the visibility-related parameters may include at least any one of color, opacity, and texture of an object included in an ultrasound image. The visibility-related parameters respectively corresponding to a plurality of object bodies may be set as predetermined values to be visually distinguished from other object bodies. In an embodiment, the visibility-related parameter of each of a plurality of object bodies may be set in units of classes setting. In other words, the visibility-related parameters of object bodies corresponding to the same class may be set to the same value. The preset data may refer to data to which the predetermined visibility-related parameter value is applied, with respect to each of a plurality of object bodies.

In other words, the ultrasound diagnostic apparatus according to an embodiment may generate pieces of preset data respectively corresponding to the plurality of object bodies by changing the visibility-related parameters respectively corresponding to the plurality of object bodies, according to the preset values, based on the area information corresponding to the three-dimensional volume data of the object and each of a plurality of object bodies.

In operation S511, the ultrasound diagnostic apparatus may render preset three-dimensional volume data of the object and output the rendered data on the display, based on the pieces of preset data with respect to the plurality of object bodies.

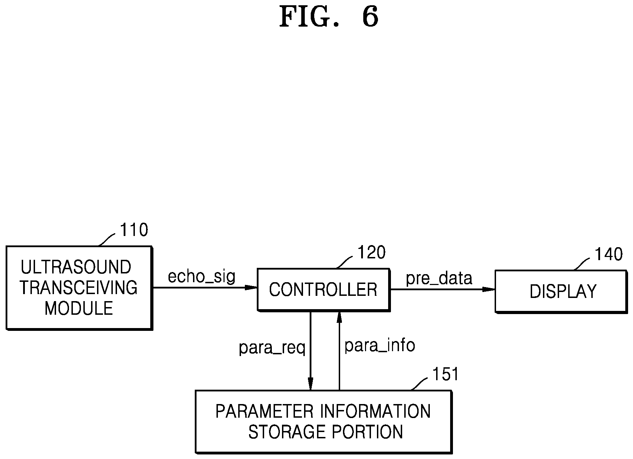

FIG. 6 is a block diagram for explaining a configuration and operation of an ultrasound diagnostic apparatus according to an embodiment.

The ultrasound diagnostic apparatus according to an embodiment may include the ultrasound transceiving module 110, the controller 120, the display 140, and a parameter information storage portion 151. The ultrasound transceiving module 110, the controller 120, and the display 140 may be the same configurations as the ultrasound transceiving module 110, the controller 120, and the display 140 of FIG. 1, respectively. The parameter information storage portion 151 may be a configuration included in the storage portion 150 of FIG. 1.

The ultrasound transceiving module 110 may transmit an ultrasound signal to the object and receive an echo signal echo_sig reflected from the object. The ultrasound transceiving module 110 may transmit the echo signal echo_sig received from the object to the controller 120.

The controller 120 may generate three-dimensional volume data of the object based on the echo signal echo_sig received from the ultrasound transceiving module 110. In this state, the ultrasound diagnostic apparatus may extract area information about each of a plurality of object bodies from the three-dimensional volume data of the object. The area information may mean information about an area in the object occupied by each of the plurality of object bodies included in the object. The three-dimensional volume data of the object may include visibility-related parameters for rendering image information corresponding to a plurality of object bodies to output through the display 140. In detail, in an embodiment, the controller 120 may generate pieces of three-dimensional volume data of the object based on the echo signal echo_sig received from the ultrasound transceiving module 110. The controller 120 may change visibility-related parameters included in the three-dimensional volume data corresponding to each of the plurality of object bodies included in the object, according to the predetermined value.

In other words, the controller 120 may generate pieces of preset data pre_data corresponding to each of the plurality of object bodies by changing the visibility-related parameters included in each of the pieces of the plurality of three-dimensional volume data. In this state, the predetermined values for the visibility-related parameters corresponding to each of the plurality of object bodies may be stored in the parameter information storage portion 151. According to an embodiment, the predetermined values may be set to be identical for each class. The controller 120 may provide the parameter information storage portion 151 with a preset value request signal para_req to set the visibility-related parameter for each class. The parameter information storage portion 151 may provide the controller 120 with preset information para_info including visibility-related parameter information for each class in response to the preset value request signal para_req. The controller 120 may provide the display 140 with the generated preset data pre_data.

The display 140 may receive the plurality of pieces of preset data pre_data from the controller 120, render the preset three-dimensional volume data of the object based on the plurality of pieces of preset data pre_data, and output the rendered data to the outside.

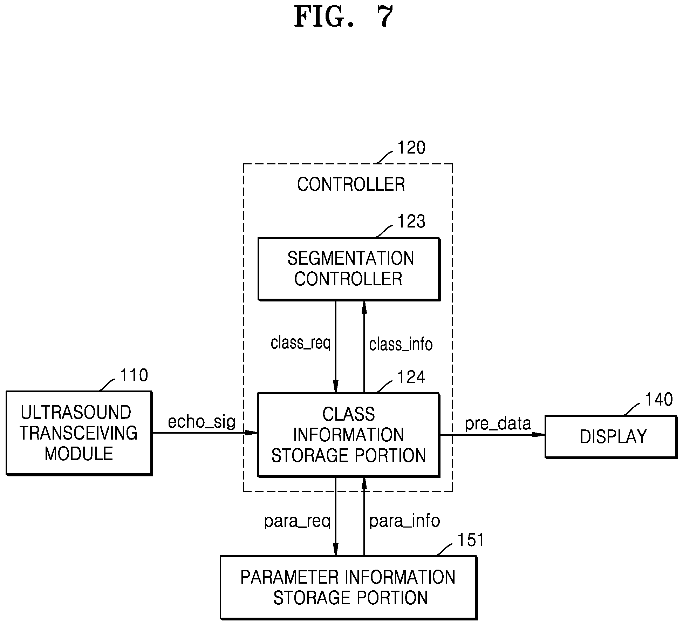

FIG. 7 is a block diagram for explaining a configuration and operation of an ultrasound diagnostic apparatus according to another embodiment.

Referring to FIG. 7, the controller 120 described with reference to FIG. 6 may further include a segmentation controller 123 and a class information storage portion 124.

The segmentation controller 123 may control the ultrasound diagnostic apparatus according to an embodiment to perform an operation of segmenting and detecting a plurality of object bodies included in an object and an operation of classifying the plurality of object bodies according to a preset class, as described with reference to FIG. 4. In an embodiment, the segmentation controller 123 may learn, based on a preset algorithm, to which class among a plurality of classes a specific part of the body, an organ, tissue, a fetus, or the like corresponds. In this state, the information stored in the class information storage portion 124 may be referred to.

The segmentation controller 123 may convert a learning result into a database and store the database in a separate storage space (not shown) included in the segmentation controller 123. The segmentation controller 123 may segment and detect each of the plurality of object bodies from three-dimensional volume data of the object, based on the learning result. In an embodiment, the segmentation of each of the plurality of object bodies from three-dimensional volume data of the object may mean information about an area in the object occupied by each of the plurality of object bodies included in the object.

The segmentation controller 123 may classify each of the plurality of object bodies according to a preset class. The segmentation controller 123 may provide the class information storage portion 124 with a class information request signal class_req for requesting information about a class to which the detected object body corresponds, to classify the plurality of object bodies. The class information storage portion 124 may provide the segmentation controller 123 with class information class_info corresponding to the detected object body that is requested by the segmentation controller 123. The segmentation controller 123 may classify each of a plurality of object bodies according to a preset class, based on the class information class_info received from the class information storage portion 124.

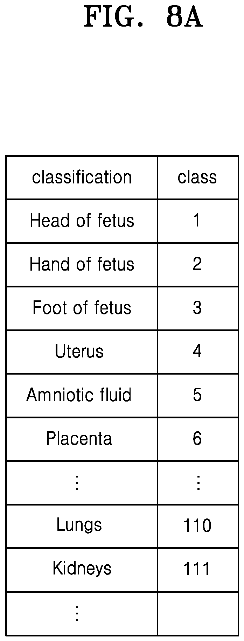

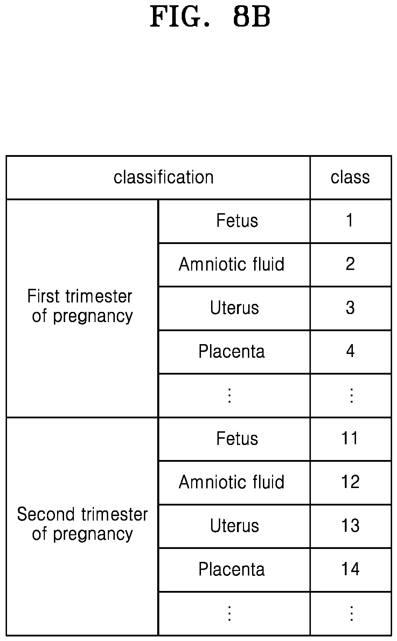

FIGS. 8A and 8B are tables for explaining a class information storage portion according to an embodiment. The class information storage portion may have the same configuration as the class information storage portion 124 of FIG. 7.

Referring to FIG. 8A, the class information storage portion may include information about a plurality of object bodies and information about a class value corresponding to each of a plurality of object bodies. Referring to FIG. 8A, the head of a fetus may correspond to Class 1. The hand of a fetus may correspond to Class 2. The foot of a fetus may correspond to Class 3. The uterus may correspond to Class 4. The amniotic fluid may correspond to Class 5. The placenta may correspond to Class 6. The lungs may correspond to Class 110. The kidneys may correspond to Class 111.

Referring to FIG. 8B, the class information storage portion may set classes for a plurality of object bodies by the pregnant woman's trimester of pregnancy. Referring to FIG. 8B, the fetus, the amniotic fluid, the uterus, and the placenta of a pregnant woman's first trimester of pregnancy may correspond to Class 1, Class 2, Class 3, and Class 4, respectively. The fetus, amniotic fluid, uterus, and placenta of a pregnant woman's second trimester of pregnancy may correspond to Class 11, Class 12, Class 13, and Class 14, respectively.

In an embodiment, the object bodies corresponding for the respective classes may be segmented and set based on clinical classification.

For example, the body of a fetus in the pregnant woman's first trimester of pregnancy may be segmented into head, neck, face, spine, chest, heart, abdomen, abdominal wall, extremities, placenta, and cord, and different classes may be set to the segmented object bodies.

For example, the body of a fetus in the pregnant woman's second trimester of pregnancy may be segmented into head, face, neck, chest/heart, abdomen, skeletal, umbilical cord, and genitalia, and different classes may be set to the segmented object bodies. The object body corresponding to each class may be variously set according to the purpose of use of the ultrasound diagnostic apparatus or the user's intention, and is not limited by the embodiment of the present disclosure described with reference to FIG. 8A or 8B.

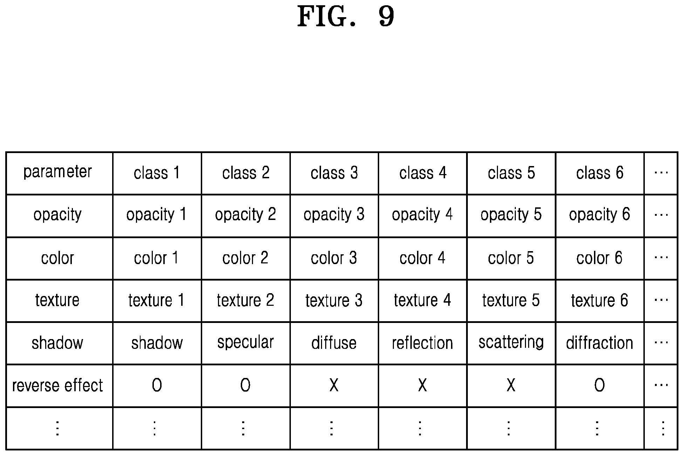

FIG. 9 is a flowchart for explaining a parameter information storage portion according to an embodiment. The parameter information storage portion may have the same configuration as the class information storage portion 124 of FIG. 7.

Referring to FIG. 9, the parameter information storage portion may include information about a plurality of classes and various pieces of visibility-related parameter information corresponding to the plurality of classes. For example, the visibility-related parameters may include opacity, color, a texture, a light effect, and a reverse effect, and the like. In an embodiment, each of the visibility-related parameters may be set by class. In an embodiment, the visibility-related parameters may be set to be different from each other by class. In another embodiment, each of the visibility-related parameters may be set to be identical for some classes.

In FIG. 9, the opacity may refer to a degree that a plurality of object bodies corresponding to the respective classes are displayed opaquely on the display. In an embodiment, the opacity values (opacity 1, opacity 2, opacity 3, opacity 4, opacity 5, and opacity 6) corresponding to the respective classes may correspond to any one value of 0% to 100%. Assuming that a first object body and a second object body included in the first object body exist, when the opacity value of the first object body is 100%, the second object body included in the first object body may be displayed as invisible by being covered by the surface of the first object body. When the opacity value of the first object body is 0%, the first object body is displayed to be completely transparent so that the first object body may not be seen and only the second object body may be displayed to be seen on the display. When the transparency values of the first object body and the second object body are each any one of medium values between 0% and 100%, the first object body and the second object body may be displayed to be overlapped each other on the display.

The color may refer to a color of each of the plurality of object bodies corresponding to the respective classes displayed on the display. The color of each of the plurality of object bodies corresponding to the respective classes may be set to any one color among various colors.

The texture may refer to a surface state of each of the plurality of object bodies displayed on the display. In an embodiment, the ultrasound diagnostic apparatus may store the properties of each of a plurality of texture values (texture 1, texture 2, texture 3, texture 4, texture 5, and texture 6). For example, a first texture value (texture 1) may be set to a smooth surface, and a second texture value (texture 2) may be set to a rough surface. In order for the first texture value (texture 1) to be set to a smooth surface, various visibility-related parameters may be adjusted. Likewise, in order for the second texture value (texture 2) to be set to a rough surface, various visibility-related parameters may be adjusted. For example, when the texture of Class 1 is set to texture 1, the surfaces of the object bodies corresponding to Class 1 may be displayed as smooth surfaces on the display. When Class 2 is set to texture 2, the surfaces of the object bodies corresponding to Class 2 may be displayed as rough surfaces on the display. The texture may be variously set without being limited to the smooth texture and the rough texture. As different texture values are set to the classes, when a plurality of object bodies are displayed together on the display, each of the plurality of object bodies may be effectively distinguished from the object bodies different from each other.

The parameter information storage portion may store visibility-related parameters (shadow, specular, diffuse, reflection, scattering, and diffraction) related to the light effect in units of classes.

The parameter information storage portion may store, in units of classes, visibility-related parameters (ambient light, specular light, and diffuse light) for the properties of a light source irradiating the object body, in relation to the light effect represented through the display.

The parameter information storage portion may store, in units of classes, visibility-related parameters for the intensity (light intensity) and direction (light direction) of light emitted to the object body, in relation to the light effect represented through the display.

The parameter information storage portion may store information about whether the reverse effect is applied to each of classes. The reverse effect may refer to an effect that the opacity of a specific object body displayed on the display is reversed. For example, in a case in which the opacity of the first object body is set to 0, when the reverse effect is applied, the opacity of the first object body displayed on the display may correspond to 100. For example, in a case in which the opacity of the second object body is set to 1, when the reverse effect is applied, the opacity of the second object body displayed on the display may correspond to 99. For example, in a case in which the opacity of the third object body is set to 10, when the reverse effect is applied, the opacity of the third object body displayed on the display may correspond to 90.

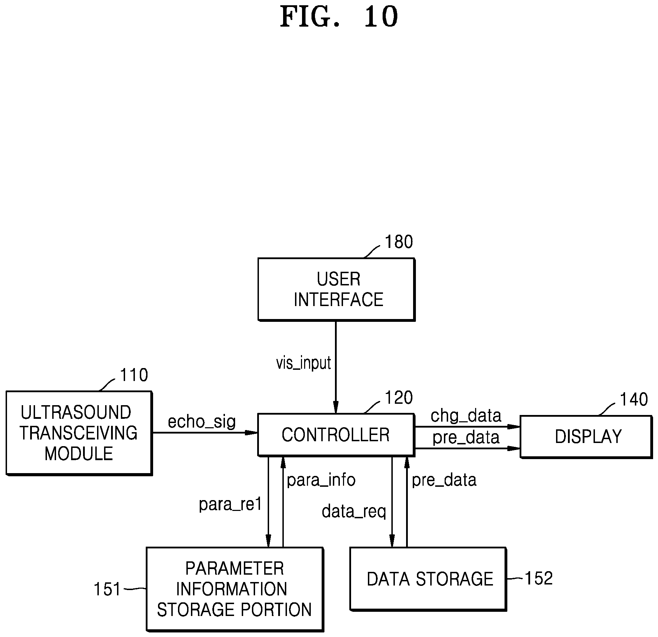

FIG. 10 is a block diagram for explaining a configuration and operation of an ultrasound diagnostic apparatus according to another embodiment. Descriptions on the same configurations as those described with reference to FIG. 6 among the configurations disclosed in FIG. 10, and the operations thereof, are omitted.

Referring to FIG. 10, the ultrasound diagnostic apparatus according to an embodiment may include a user interface 180. The user interface 180 may include an interface to change visibility-related parameters for a plurality of object bodies displayed on the display. The user interface 180 may be implemented in the form of a touch screen included in the display 140 as described with reference to FIG. 2. The user interface 180 may be implemented in the form of the sub-display 122 as described with reference to (a) and (b) of FIG. 2. The user interface 180 may include a control panel to adjust visibility-related parameters of the object bodies displayed on the display. A user using the ultrasound diagnostic apparatus may adjust, through the user interface 180, visibility-related parameters of the object bodies displayed on the display the user interface 180.

In an embodiment, the user interface 180 may provide the controller 120 with a change input signal vis_input including changes of visibility-related parameters. The controller 120 may generate changed data chg_data, by changing visibility-related parameters for the plurality of pieces of preset data pre_data according to the changes in response to the change input signal vis_input. The controller 120 may provide the changed data chg_data to the display. The display 140 may render the three-dimensional volume data of the object changed based on the changed data chg_data received from the controller 120 and output the rendered data.

Although not disclosed in FIG. 6, the ultrasound diagnostic apparatus according to an embodiment may further include a data storage portion 152 to store the plurality of pieces of preset data pre_data. When generating the preset data pre_data, the controller 120 may provide the generated preset data pre_data not only to the display 140, but also to the data storage portion 152. The data storage portion 152 may store the preset data pre_data. When receiving the change input signal vis_input from the user interface 180, the controller 120 may provide a preset data request signal data_req to the data storage portion 152. The data storage portion 152 may provide the controller 120 with the preset data pre_data corresponding to the request by the controller 120, in response to the preset data request signal data_req. The controller 120 may generate the changed data chg_data by converting the preset data pre_data received from the data storage portion 152 according to changes received from the user interface 180.

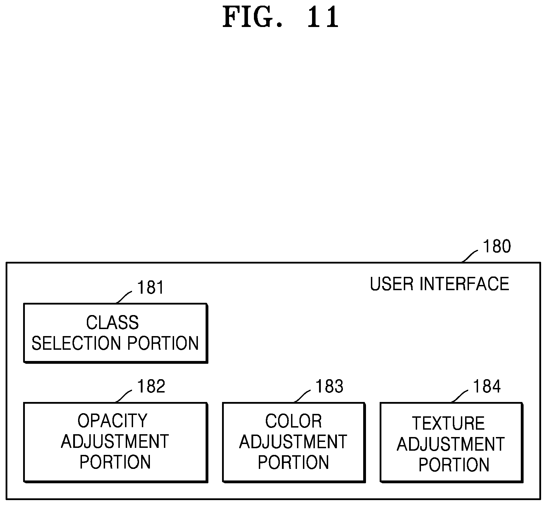

FIG. 11 is a block diagram for explaining a user interface according to an embodiment.

The user interface 180 may include an interface to select a specific one of a plurality of object bodies and at least one interface to change visibility-related parameters of a selected object body.

For example, referring to FIG. 11, the user interface 180 may include a class selection portion 181, an opacity adjustment portion 182, a color adjustment portion 183, and a texture adjustment portion 184.

A user may select one of a plurality of classes through the class selection portion 181. In this case, one or a plurality of object bodies corresponding to each class may be selected.

The user may adjust the opacity of object bodies corresponding to the selected class, through the opacity adjustment portion 182.

The user may adjust the colors of object bodies corresponding to the selected class, through the color adjustment portion 183.

The configurations included in the user interface 180 may be implemented in various methods, without being limited to the configurations described with reference to FIG. 11 as an example. The user interface 180 may include configurations to adjust not only opacity, color, and texture, but also various visibility-related parameters. For example, the user interface 180 may include a configuration to adjust a light effect or a reverse effect.

The user interface 180 may provide the controller with changes with respect to visibility parameters input by the user.

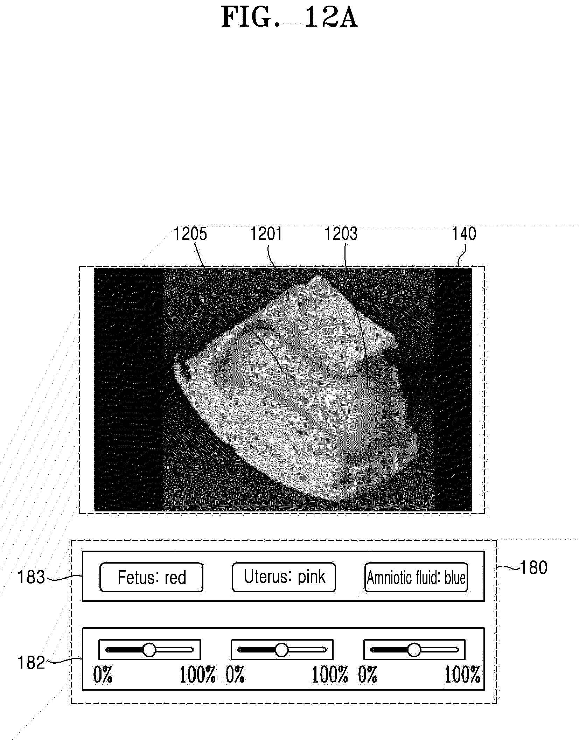

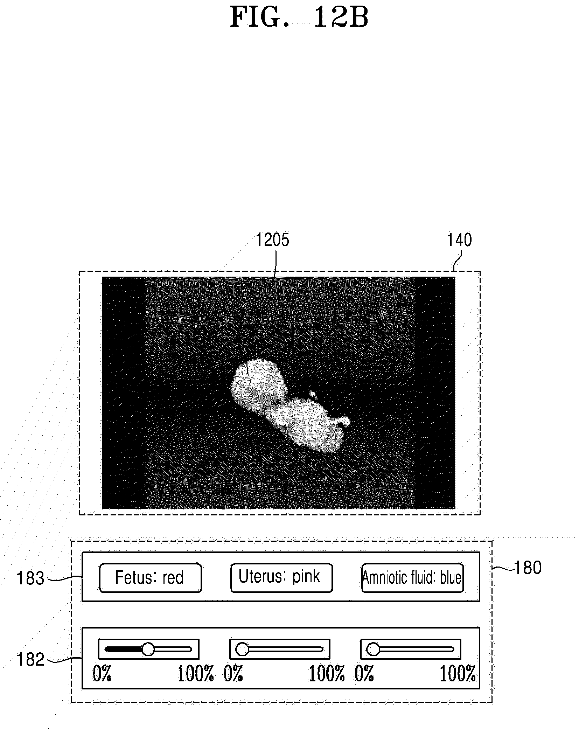

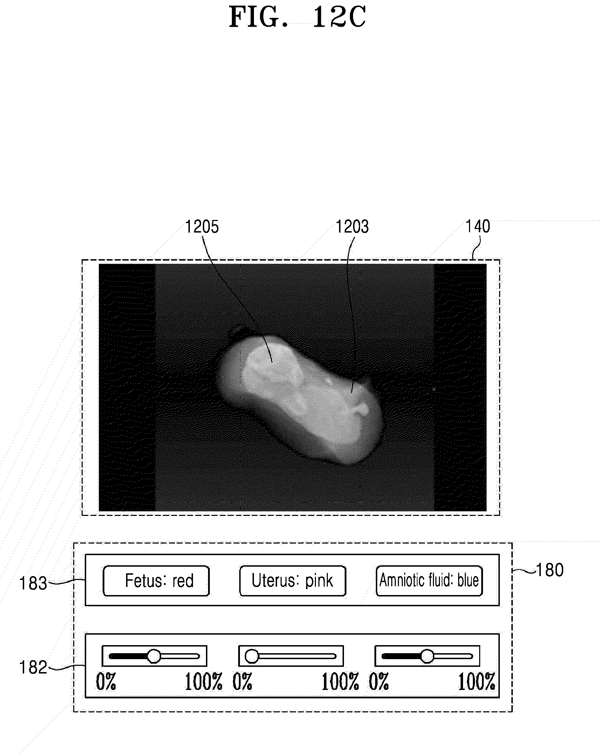

FIGS. 12A, 12B, and 12C are views for explaining content displayed on a display of an ultrasound diagnostic apparatus according to an embodiment.

In FIGS. 12A, 12B, and 12C, the display 140 may display an image of at least any one of a uterus 1201, an amniotic fluid 1203, and a fetus 1205. The display 140 may be the same configuration as the display 140 described with reference to FIGS. 6 to 7 and 10. The user interface 180 may include the color adjustment portion 183 and the opacity adjustment portion 182. The user interface 180, the color adjustment portion 183, and the opacity adjustment portion 182 may be the same configuration as the user interface 180, the color adjustment portion 183, and the opacity adjustment portion 182 described with reference to FIG. 11.

Referring to FIG. 12A, the 'fetus' may be set to 'red' through the color adjustment portion 183. In this state, the color of the fetus 1205 output through the display 140 may be red. The 'uterus' may be set to 'pink' through the color adjustment portion 183. In this state, the color of the uterus 1201 output through the display 140 may be pink. The 'amniotic fluid' may be set to 'blue' through the color adjustment portion 183. In this state, the color of the amniotic fluid 1203 output through the display 140 may be blue.

Referring to FIG. 12A, the opacity of the uterus 1201, the amniotic fluid 1203, and the fetus 1205 displayed on the display 140 may be adjusted through the opacity adjustment portion 182. The opacity adjustment portion 182 of FIGS. 12A, 12B, and 12C may include three sub-adjustment portions to adjust the opacity of the uterus 1201, the amniotic fluid 1203, and the fetus 1205. For example, in the opacity adjustment portion 182, a left sub-adjustment portion may be to adjust the opacity of the fetus 1205. For example, in the opacity adjustment portion 182, a middle sub-adjustment portion may be to adjust the opacity of the uterus 1201. For example, in the opacity adjustment portion 182, a right sub-adjustment portion may be to adjust the opacity of the amniotic fluid 1203. Referring to FIG. 12A, as the opacity of the fetus 1205, the uterus 1201, and the amniotic fluid 1203 has a medium value between 0% and 100%, as described with reference to FIG. 9, the fetus 1205, the uterus 1201, and the amniotic fluid 1203 may be displayed in an overlapping state on the display 140.

Referring to FIG. 12B, in an embodiment, the 'fetus', the 'uterus,' and the 'amniotic fluid' may be set to 'red', 'pink,' and 'blue,' respectively, through the color adjustment portion 183.

Referring to FIG. 12B, in an embodiment, the opacity of the fetus 1205 may be set to a medium value between 0% and 100%, and the opacity of the uterus 1201 and the amniotic fluid 1203 may be set to 0%, through the opacity adjustment portion 182. Accordingly, as the uterus 1201 and the amniotic fluid 1203 are displayed to be completely transparent, the uterus 1201 and the amniotic fluid 1203 are not seen through the display 140, and only the fetus 1205 may be displayed to be seen on the display 140.

Referring to FIG. 12C, in an embodiment, the 'fetus', the 'uterus,' and the 'amniotic fluid' may be set to 'red', 'pink,' and 'blue,' respectively, through the color adjustment portion 183.

Referring to FIG. 12C, in an embodiment, the opacity of the fetus 1205 and the amniotic fluid 1203 may be set to a medium value between 0% and 100%, and the opacity of the uterus 1201 may be set to 0% through the opacity adjustment portion 182. Accordingly, as the uterus 1201 is displayed to be completely transparent, the uterus 1201 is not seen through the display 140, and only the fetus 1205 and the amniotic fluid 1203 may be displayed in an overlapping state on the display 140.

As described with reference to FIGS. 12A to 12C, specific object bodies in the human body are assigned with different classes, and then, visibility parameters are adjusted by class so that disease diagnosis through the ultrasound diagnostic apparatus may be effectively performed.

For example, after different classes are assigned to the uterus, the fetus, and the placenta, by adjusting the visibility-related parameters for each class, for example, semi-transparency, color, and the like, a relative locational relationship of the uterus, the fetus, and the placenta may be clearly identified. Accordingly, it may be possible to effectively diagnose the presence or absence of the pregnant woman's placenta previa and the premature detachment state of the placenta. The placenta previa may mean that the placenta is attached to the bottom of the uterus instead of the top thereof. The premature detachment may refer to the premature separation of a normally positioned placenta from the uterus wall.

In an embodiment, by assigning different classes to the placenta and other organs and then adjusting the visibility-related parameters for each class, it may be possible to diagnose whether the placenta has penetrated into other tissues during the pregnancy cycle. For example, by assigning different classes to the placenta and the pregnant woman's bladder and then adjusting the visibility-related parameters for each class, it may be possible to determine the extent to which the placenta has penetrated into the lumen of the pregnant woman's bladder.

In an embodiment, by assigning different classes to the placenta and other organs and then adjusting the visibility-related parameters for each class, it may be possible to diagnose whether placenta remnants exist in the uterus after an abortion operation.

In an embodiment, by assigning different classes to the umbilical cord and other organs and then adjusting the visibility-related parameters for each class, it may be possible to diagnose whether the umbilical cord is abnormal or not. For example, it may be possible to diagnose morphological abnormality such as twisting of the umbilical cord and the like.

In an embodiment, by assigning different classes to the face and other body parts and then adjusting the visibility-related parameters for each class, it may be possible to easily diagnose diseases related to the shape of the face. For example, morphological diagnosis of cleft lip related diseases may be effectively performed.

In an embodiment, by assigning different classes to the spine of fetus and other body configurations and then adjusting the visibility-related parameters for each class, it may be possible to effectively diagnose the posture and position of the fetus.

In an embodiment, by assigning different classes to the spine of fetus and other body configurations and then adjusting the visibility-related parameters for each class, diseases related to the spine may be easily diagnosed. For example, the degree of development of the central nervous system, such as spinal bifida and the like, may be easily diagnosed.

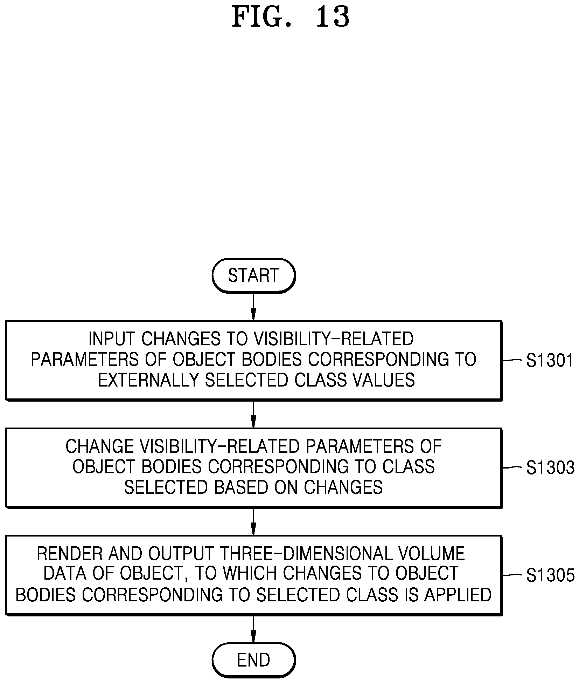

FIG. 13 is a flowchart for explaining content in which an ultrasound diagnostic apparatus according to an embodiment outputs an ultrasound image that reflects changes.

In operation S1301, changes to the visibility-related parameters of object bodies corresponding to the class selected by the user may be input through the user interface included in the ultrasound diagnostic apparatus.

In operation S1303, the ultrasound diagnostic apparatus may change the visibility-related parameters of object bodies corresponding to the class selected based on the changes.

In operation S1305, the ultrasound diagnostic apparatus may render and output three-dimensional volume data of the object, to which changes to the object bodies corresponding to the selected class is applied.

As described above, the disclosed embodiments are described with reference to the accompanying drawings. The above descriptions of the disclosure is for an example, and it will be understood that one of ordinary skill in the art to which the disclosure pertains can easily modify the disclosure into other detailed form without changing the technical concept or essential features of the disclosure. Thus, the above-described embodiments are exemplary in all aspects and should not be for purposes of limitation.

## Claims

1. An ultrasound diagnostic apparatus comprising: an ultrasound signal transceiving module configured to transmit an ultrasound signal to an object and receive an ultrasound echo signal reflected from the object;
a controller configured to extract area information that is information about an area in the object occupied by each of the plurality of object bodies included in the object, based on the echo signal received from the ultrasound signal transceiving module, and generate a plurality of pieces of preset data corresponding to each of the plurality of object bodies by changing visibility-related parameters corresponding to an area occupied by each of the plurality of object bodies according to a predetermined value, based on the area information;
a parameter information storage portion configured to store information about the predetermined value and provide the information about the predetermined value to the controller in response to a request by the controller; and
a display configured to receive the plurality of pieces of preset data from the controller and render and output preset three-dimensional volume data of the object based on the plurality of pieces of preset data.

2. The ultrasound diagnostic apparatus of claim 1, wherein the controller is further configured to: when the echo signal is received, generate the three-dimensional volume data of the object; extract area information corresponding to each of the plurality of object bodies from the three-dimensional volume data of the object, by using a semantic segmentation method; perform a classification operation to match each of the plurality of object bodies to any one of a plurality of predetermined classes, based on the extracted area information corresponding to each of the plurality of object bodies; and generate a plurality of pieces of preset data corresponding to each of the plurality of object bodies.

3. The ultrasound diagnostic apparatus of claim 2, wherein
the plurality of predetermined classes correspond to any one of a plurality of organs included in a human body or a fetus, and
the controller comprises
a class information storage portion comprising information about the plurality of predetermined classes and information about a plurality of organs and a fetus corresponding to each of the plurality of predetermined classes.

4. The ultrasound diagnostic apparatus of claim 2, wherein
the parameter information storage portion comprises information about the plurality of classes and information about the predetermined value corresponding to each of the plurality of classes.

5. The ultrasound diagnostic apparatus of claim 2, wherein
the visibility-related parameters comprise at least any one of color, opacity, and texture, and the parameter information storage portion is further configured to store information about a predetermined value corresponding to each of the visibility-related parameters for each class.

6. The ultrasound diagnostic apparatus of claim 2, further comprising
a user interface configured to change the visibility-related parameters,
the user interface comprises at least any one of a color adjustment portion, an opacity adjustment portion, and a texture adjustment portion, and
when changes to the visibility-related parameters are input through the user interface, the controller is further configured to change visibility-related parameters for pieces of preset data corresponding to each of the plurality of object bodies according to the changes and provided pieces of changed data to the display.

7. The ultrasound diagnostic apparatus of claim 6, wherein the user interface further comprises a class selection portion configured to select any one of the plurality of classes,
wherein, when changes to the visibility-related parameters are input through the user interface, the controller is further configured to change visibility-related parameters included in pieces of preset data of the plurality of object bodies corresponding to a class selected by the class selection portion among the plurality of object bodies according to a user's input.

8. The ultrasound diagnostic apparatus of claim 6, wherein the display is further configured to, when receiving the pieces of changed data from the controller, render and output the three-dimensional volume data of the object changed based on the changed data.

9. The ultrasound diagnostic apparatus of claim 6, wherein the controller further comprises a data storage portion configured to store a plurality of pieces of preset data corresponding to each of the plurality of object bodies, and
when receiving the changes from the user interface, is further configured to receive the plurality of preset data from the data storage portion and change visibility-related parameters included in the plurality of pieces of preset data received from the data storage portion, according to the changes.

10. The ultrasound diagnostic apparatus of claim 3, wherein the controller is further configured to match each of the plurality of object bodies to any one of the plurality of predetermined classes, based on the information about the plurality of predetermined classes and the information about a plurality of organs and a fetus corresponding to each of the plurality of predetermined classes included in the class information storage portion.

11. An operation method of an ultrasound diagnostic apparatus, the operating method comprising:
transmitting an ultrasound signal to an object and receiving an ultrasound echo signal reflected from the object;
extracting area information that is information about an area in the object occupied by each of the plurality of object bodies included in the object, based on the echo signal; changing visibility-related parameters corresponding to an area occupied by each of the plurality of object bodies according to a predetermined value based on the area information and generating a plurality of pieces of preset data corresponding to each of the plurality of object bodies; and
rendering and outputting preset three-dimensional volume data of the object based on the plurality of pieces of preset data.

12. The operation method of claim 11, further comprising
storing information about the predetermined value in a parameter information storage portion included in the ultrasound diagnostic apparatus.

13. The operation method of claim 11, wherein the extracting of the area information comprises:
generating the three-dimensional volume data of the object based on the echo signal;
extracting area information corresponding to each of the plurality of object bodies by using a semantic segmentation method;
matching each of the plurality of object bodies to any one of a plurality of predetermined classes, based on the extracted area information corresponding to each of the plurality of object bodies; and
generating a plurality of pieces of preset data corresponding to each of the plurality of object bodies.

14. The operation method of claim 13, wherein
the plurality of predetermined classes correspond to any one of a plurality of organs included in a human body or a fetus,
the operation method further comprising storing information about the plurality of classes and information about a plurality of organs and a fetus corresponding to each of the plurality of predetermined classes in a class information storage portion in the ultrasound diagnostic apparatus.

15. The operation method of claim 12, wherein the storing of the information about the predetermined value in the parameter information storage portion comprises storing information about the predetermined value corresponding to each of the plurality of classes.

16. The operation method of claim 13, wherein
the visibility-related parameters comprise at least any one of color, opacity, and texture, and the storing of the information about the predetermined value in the parameter information storage portion included in the ultrasound diagnostic apparatus comprises storing information about a predetermined value corresponding to each of the visibility-related parameters for each class.

17. The operation method of claim 16, further comprising:
when changes to the visibility-related parameters are input from outside, changing visibility-related parameters with respect to pieces of preset data corresponding to each of the plurality of object bodies, according to the changes; and
rendering and outputting three-dimensional volume data of the object changed based on the changed data.

18. The operation method of claim 17, wherein the changing of the visibility-related parameters further comprises:
selecting any one of the plurality of classes according to a user's input; and
changing visibility-related parameters included in pieces of preset data of the plurality of object bodies corresponding to a class selected from among the plurality of object bodies.

19. The operation method of claim 11, further comprising
storing a plurality of pieces of preset data corresponding to each of the plurality of object bodies in a data storage portion included in the ultrasound diagnostic apparatus.

20. The operation method of claim 14, wherein the matching of the each of the plurality of detected object bodies to any one of a plurality of predetermined classes comprises matching each of the plurality of object bodies to any one of the plurality of predetermined classes, based on the information about the plurality of predetermined classes and the information about a plurality of organs and a fetus corresponding to each of the plurality of predetermined classes stored in the class information storage portion.
